# EUROPEAN PATENT SPECIFICATION

(11) **EP 2 936 462 B1**
(45) Date of publication and mention of the grant of the patent: **05.06.2019**
(21) Application number: 12890472.9
(22) Date of filing: 20.12.2012
(51) Int. Cl.: G08B 1/08, G06Q 50/22

(54) **SYSTEM AND METHOD FOR MANAGING PATIENT ENVIRONMENT**
SYSTEM UND VERFAHREN ZUR VERWALTUNG EINER PATIENTENUMGEBUNG
SYSTÈME ET PROCÉDÉ DE GESTION D'ENVIRONNEMENT DE PATIENT

(43) Date of publication of application: 28.10.2015
(73) Proprietor: Schneider Electric Buildings, LLC, Palatine, IL 60067-7399 (US)
(72) Inventor: ROSEBRAUGH, Warren, Dale, Salem, NH 03079 (US)
(74) Representative: Murgitroyd & Company
(86) International application number: PCT/US2012/070853
(87) International publication number: WO 2014/098861

(56) References cited:
- US-A1- 2003 227 386
- US-A1- 2005 093 694
- US-A1- 2007 194 939
- US-A1- 2011 001 605
- US-A1- 2011 112 854

## Description

### BACKGROUND

### Technical Field

The technical field of this disclosure relates generally to building control systems and, more particularly, to systems and methods that automatically vary environmental controls responsive to occupant tracking.

### Background Discussion

Energy conservation has become a benchmark for evaluating any system that consumes power. For systems that consume large volumes of power, any energy savings is deemed valuable. Environment control systems (e.g., air conditioning, heat, etc.) typically consume large amounts of power to provide a comfortable and/or consistent environment, for example, in a user's home, office, or other building subject to control. In a patient care setting, environmental control can even assist in treatment and patient recuperation. In some examples of patient care, specific levels of environmental control are mandated for compliance purposes. Some conventional systems for managing environmental control provide for some reduction in energy consumption using static programs and/or settings.

For example, building management systems ("BMS") exist that facilitate managing an environment within a building and can control temperature, carbon dioxide levels, and humidity within a building. Most BMS systems also provide for heating and cooling control, and can manage the systems that distribute air throughout the building (for example by operating fans or opening/closing dampers). US 2011/0112854 shows an environmental control system based on rooms being scheduled to be in use or empty.

### SUMMARY

The invention is defined in the appended claims.

It is realized that improvements over conventional environment control systems can provide significant savings, for example, in terms of energy consumption. In particular, environment control systems can integrate with other systems and/or building management control to provide significant reduction in power consumption by minimizing the use of environmental control. In a patient care environment, standardized systems exist for controlling patient admissions, discharges, and transfers. According to one aspect, an environment control system can be integrated with patient administration systems (e.g., admission, discharge, and transfer systems or "ADT" systems) to implement energy saving algorithms. The energy saving algorithms can be based on the presence or absence of a patient from a given room. In some embodiments, tracking of patients by bed can enable further optimization of environmental control. In further embodiments, additional patient administration systems can be integrated to provide finer tune control, and can enable configurations to manage an environment based on scheduled testing, operations, imaging, or any procedure which takes a patient out of their room.

According to another aspect, environment control systems can include integration with building management systems (including system for controlling, e.g., air, humidity, temperature, and lighting, among other options) as well as administration systems. An environmental control system can be configured to communicate with any administration and BMS systems to provide improved control over temperature settings, airflow settings, among other options.

According to one aspect, a system for environmental control of patient care environments is provided. The system comprises at least one processor operatively connected to a memory, the at least one processor when executing is configured to determine information associated with patient location for a plurality of patient rooms, establish at least one change to an environmental control parameter responsive to determining the information associated with patient location, and communicate an environment control message to a environment management subsystem configured to implement at least one change to the environment control parameter.

According to one embodiment, the system further comprises a generation component configured to generate the environment control message including the at least one change to the environmental control parameter. According to one embodiment, the system further comprises a communication component configured to receive administration messages regarding patient location. According to one embodiment, the system further comprises an interpretation component configured to capture the information associated with patient location from the administration messages. According to one embodiment, the interpretation component is configured to capture the information associated with patient location from HL7 formatted messages.

According to one embodiment, the system further comprises a state component configured to determine a state for at least one room based at least in part on the information associated with patient location. According to one embodiment, the state component is configured to determine the state according to a number of beds associated with the at least one room.

According to one embodiment, the system further comprises a control component configured to select the at least one change in the environmental control parameter based on the state for the at least one room. According to one embodiment, the control component is configured to select the at least one change in the environmental control parameter based on a time period associated with the state. According to one embodiment, the state for the at least one room includes a state for respective ones of a plurality of beds in the at least one room. According to one embodiment, the at least one change to an environmental control parameter is configured to control at least one of temperature, airflow, humidity, CO2 level, and lighting operation.

According to one embodiment, the system further comprises an administration subsystem configured to manage at least one of patient admission, discharge, and transfer. According to one embodiment, the system further comprises the environment management subsystem, wherein the environment management subsystem is configured to manage environment devices configured to provide for at least one of heating, cooling, airflow, humidifying, and lighting.

According to one aspect, a method for environmental control of patient care environments is provided. The method comprises determining, by a computer system, information associated with patient location for a plurality of patient rooms, establishing, by the computer system, at least one change to an environmental control parameter responsive to determining the information associated with patient location, and communicating, by the computer system, an environment control message to a environment management subsystem configured to implement at least one change to the environment control parameter.

According to one embodiment, the method further comprises generating, by the computer system, the environment control message including the at least one change to the environmental control parameter. According to one embodiment, the method further comprises receiving, by the computer system, administration messages regarding patient location. According to one embodiment, the method further comprises capturing the information associated with patient location from the administration messages. According to one embodiment, capturing the information associated with patient location from the administration messages includes capturing the information associated with patient location from HL7 formatted messages.

According to one embodiment, the method further comprises determining a state for at least one room based at least in part on the information associated with patient location. According to one embodiment, determining the state includes determining the state according to a number of beds associated with the at least one room. According to one embodiment, the method further comprises selecting the at least one change in the environmental control parameter based on the state for the at least one room.

According to one aspect, a non-transitory computer readable medium is provided. The non- transitory computer readable medium having stored thereon sequences of instruction for environmental control in a patient care environment including instructions that cause at least one processor of a computer system to determine information associated with patient location for a plurality of patient rooms, establish at least one change to an environmental control parameter responsive to determining the information associated with patient location, and communicate an environment control message to a environment management subsystem configured to implement at least one change to the environment control parameter.

According to one embodiment, the at least one processor is caused to generate the environment control message including the at least one change to the environmental control parameter. According to one embodiment, the at least one processor is caused to receive administration messages regarding patient location. According to one embodiment, the at least one processor is caused to capture information associated with patient location from the administration messages. According to one embodiment, capturing the information associated with patient location from the administration messages includes capturing the information associated with patient location from HL7 formatted messages.

According to one embodiment, the at least one processor is caused to determine a state for at least one room based at least in part on the information associated with patient location. According to one embodiment, determining the state includes determining the state according to a number of beds associated with the at least one room. According to one embodiment, the at least one processor is caused to select the at least one change in the environmental control parameter based on the state for the at least one room.

Other aspects, embodiments and advantages of these exemplary aspects and embodiments, are discussed in detail below. Moreover, it is to be understood that both the foregoing information and the following detailed description are merely illustrative examples of various aspects and embodiments, and are intended to provide an overview or framework for understanding the nature and character of the claimed aspects and embodiments. Any embodiment disclosed herein may be combined with any other embodiment. References to "an embodiment," "an example," "some embodiments," "some examples," "an alternate embodiment," "various embodiments," "one embodiment," "at least one embodiment," "this and other embodiments" or the like are not necessarily mutually exclusive and are intended to indicate that a particular feature, structure, or characteristic described in connection with the embodiment may be included in at least one embodiment. The appearances of such terms herein are not necessarily all referring to the same embodiment or example.

### BRIEF DESCRIPTION OF DRAWINGS

Various aspects of at least one embodiment are discussed below with reference to the accompanying figures, which are not intended to be drawn to scale. The figures are included to provide an illustration and a further understanding of the various aspects and embodiments, and are incorporated in and constitute a part of this specification, but are not intended as a definition of the limits of any particular embodiment. The drawings, together with the remainder of the specification, serve to explain principles and operations of the described and claimed aspects and embodiments. In the figures, each identical or nearly identical component that is illustrated in various figures is represented by a like numeral. For purposes of clarity, not every component may be labeled in every figure. In the figures:
FIG. 1 is a block diagram of an example environmental control system;
FIG. 2 is a block diagram of an example environmental control system;
FIG. 3 is a flow diagram illustrating a process for controlling environmental conditions responsive to patient location;
FIG. 4 is a flow diagram illustrating a process for defining environmental changes; and
FIG. 5 a schematic diagram of an exemplary computer system that may be configured to perform processes and functions disclosed herein.

### DETAILED DESCRIPTION

At least some embodiments disclosed herein include apparatus and processes for controlling environment parameters according to patient occupancy of managed rooms. In some embodiments, environment set points (e.g., temperature settings, airflow settings, lighting controls, etc.) can be maintained, modified, and/or reset based on administration information regarding patient occupancy. For example, when patients are admitted, transferred, and/or discharged an administration system can be configured to communicate this information to an environmental control system. The environmental control system can determine a state of a room (e.g., a patient's room) based on its occupancy, and use that state to control environmental settings through a BMS system.

According to one embodiment, environmental control system can communicate control messages for temperature settings based on changes in a room's state. In some examples, the environmental control system can be configured to provide for varying levels of temperature control based on a type of state change. In one implementation, types of state change can include temporary occupancy state changes where a patient is expected to return. Types of state change can also include persistent changes. For example, when a room becomes unoccupied based on discharge, the environmental control system can cause the BMS system to reset the room to a much lower temperature. The unoccupied state can also be coupled with other environment set points, including, for example, an airflow rate. The environmental control system can be configured to transition between occupied settings and unoccupied settings responsive to messages from the administration system. Unoccupied settings stored by the environmental control system can also include lighting control settings, among other options.

In another example, when a room is temporarily unoccupied the environmental control system can cause the BMS system to reset the room to another temperature setting but with a smaller degree of change relative to a persistent state change (e.g., a room unoccupied based on a discharge). The system can determine the type of change based on administration messages. In one embodiment, administration messages regarding patient admissions, transfers, and discharges can be "HL7" compliant. "HL7" refers to a known set of ANSI-accredited standards for the exchange, integration, sharing, and retrieval of electronic health information. In various embodiments, the environmental control system is configured to accept and interpret administration communications formatted according to HL7 standards. In other embodiments, the environmental control system can be configured to accept messages according to other formats in addition to, or instead of HL7 compliant messaging.

Examples of the methods and systems discussed herein are not limited in application to the details of construction and the arrangement of components set forth in the following description or illustrated in the accompanying drawings. The methods and systems are capable of implementation in other embodiments and of being practiced or of being carried out in various ways. Examples of specific implementations are provided herein for illustrative purposes only and are not intended to be limiting. In particular, acts, components, elements and features discussed in connection with any one or more examples are not intended to be excluded from a similar role in any other examples.

Also, the phraseology and terminology used herein is for the purpose of description and should not be regarded as limiting. Any references to examples, embodiments, components, elements or acts of the systems and methods herein referred to in the singular may also embrace embodiments including a plurality, and any references in plural to any embodiment, component, element or act herein may also embrace embodiments including only a singularity. References in the singular or plural form are not intended to limit the presently disclosed systems or methods, their components, acts, or elements. The use herein of "including," "comprising," "having," "containing," "involving," and variations thereof is meant to encompass the items listed thereafter and equivalents thereof as well as additional items. References to "or" may be construed as inclusive so that any terms described using "or" may indicate any of a single, more than one, and all of the described terms.

### Environmental Control System

Some embodiments implement an environmental control system that provides for and can automatically control environmental settings for managed rooms according to occupancy information. According to one embodiment, the environmental control system can be implemented in conjunction with patient administration subsystems. The environmental control system can also include and/or integrate with BMS subsystems configured to manage, for example, heating, lighting, air-conditioning, airflow, humidity, CO2 levels, among other options.

According to another embodiment, the environmental control system manages integration between the BMS system and the ADT subsystems. In one example, the environmental control system can be configured to employ HL7 protocols to implement energy savings algorithms controlling environmental parameters of patient rooms in a hospital. The environmental control system can receive HL7 messages from an ADT subsystem, determine patient locations associated with managed rooms, and deliver environmental control messages to the BMS subsystems. The control message can include new environment set points. In some examples, the environment set points can define new environment control settings (e.g., 62 degrees, number of air exchanges per hour, light patterns for on and off lighting, etc.) or define relative changes to existing set points (e.g., - 10 degrees, +10 degrees, ½ current air exchange rate, etc.). The environmental control system can be configured to communicate control messages to the BMS subsystems using known protocols (e.g., BACnet over IP - a standard for building automation and control systems), provide control messages tailored to a specific BMS system, and/or in some examples, to communicate directly with the devices that provide heating, cooling, lighting, etc.

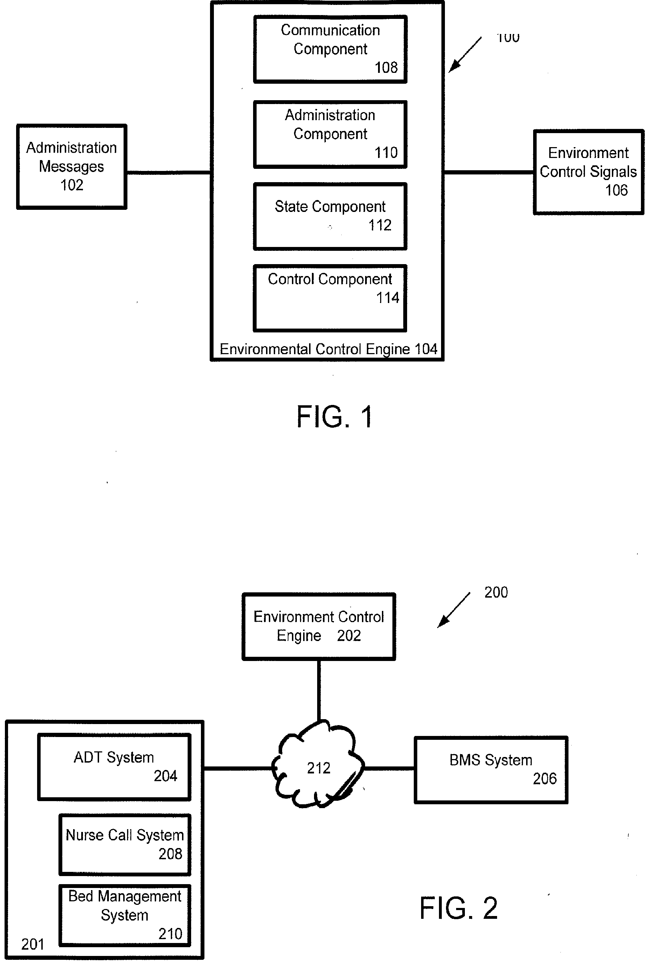

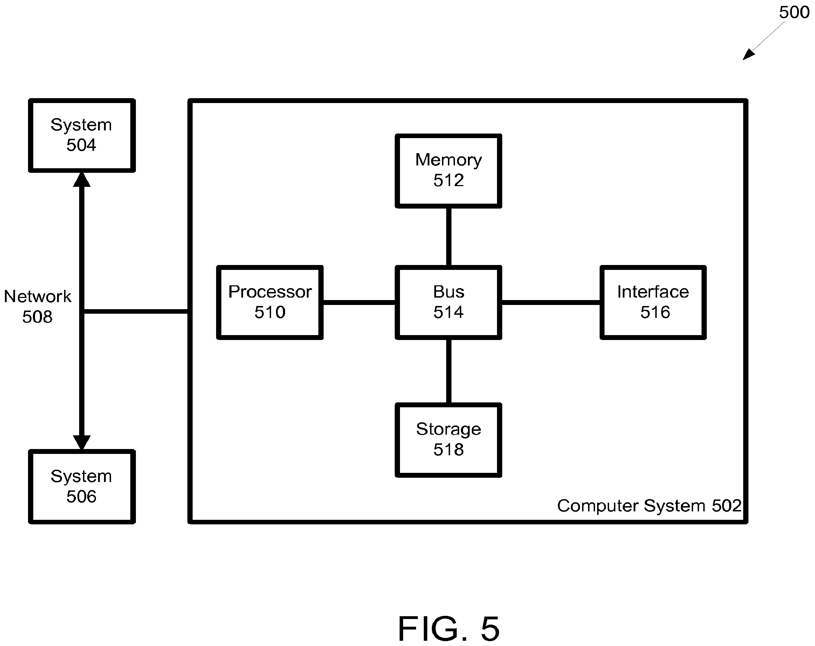

FIG. 1 illustrates one embodiment of an environment control system 100. The environment control system 100 can be configured to manage environmental parameters through integration with hospital administration subsystems and BMS subsystems, for example, using an environmental control engine 104. Elements of the system 100 can be provided using a computing system such as the computer system 500 and/or 502 described with reference to FIG. 5. For example, the environment control engine 104 can be executed on the computer system 500 and/or 502 to provide the functions and operations discussed herein. In other embodiments, the environment control engine 104 can include additional components executed on the computer system to perform specific operations. In some implementations, the system 100 and/or environment control engine 104 can be configured to communicate with existing ADT and/or BMS systems, to provide management of environment settings responsive to patient occupancy information.

As shown in FIG. 1, the environment control engine 104 receives input from an administration system associated with managing patient care. According to one embodiment, system 100 is configured to receive administration messages 102 regarding, for example, patient admission, patient transfer, and/or patient discharge. In some embodiments, the administration messages 102 can be communicated from ADT subsystems. For example, the ADT subsystems can be pre-existing and the environmental control engine 104 can be implemented on the computer systems associated with the ADT subsystem. In another example, the environment control engine 104 can be implemented on computers connected to the ADT subsystem.

In some embodiments, the ADT subsystem can be HL7 compliant, and format administration messages (e.g., 102) according to HL7 standards. According to one embodiment, the environment control engine 104 can be configured to interpret received administration messages to capture information regarding patient location, patient room assignment, patient bed assignment, and any other information regarding scheduling of a patient's location, among other options.

In one example, the environment control engine 104 can include a communication component 108 configured to receive and interpret administration messages to determine when, for example, a patient is assigned to a room and/or a bed within a room. In some embodiments, the communication component 108 can be configured to identify HL7 messages upon receipt and analyze the message to determine, for example, information regarding patient assignment, room, location, patient bed, and any schedule events for the patient (e.g., admission date, admission time, scheduled tests, scheduled procedures, discharge date, discharge time, etc.). The communication component 108 can also be configured to analyze other administration messages 102 of other formats to determine information regarding patient assignments to rooms and/or beds, and any information regarding patient location and scheduling.

According to one embodiment, the environment control engine 104 can be configured to determine a state for a room responsive to interpreting administration messages. In one example, the communication component 108 is configured to capture information from the administration messages 102 and pass the occupancy information to a state component 112 configured to determine a state associated with a given room. In some embodiments, the communication component 108 is configured to analyze administration communications for occupancy information and store that information in a data storage location (e.g., a database). The state component 112 can access stored information and analyze the stored information to determine if a given room is, or is scheduled to be, occupied. In some embodiments, the administration information can be stored in association with room records, and a state describing an occupancy status for the room can also be associated with a room record.

The state component 112 can also be configured to determine occupancy of each bed within a room and enable environmental control based on occupancy status of each bed within a given room.

The number of rooms in a managed facility (e.g., hospital) and the number of beds within each room can be configured on the environmental control system at initialization, installation, or other time. In one example, an administration component (e.g., administration component 110, discussed in greater detail below) can be configured to accept information regarding the number of rooms and the number of beds for each respective room. In some embodiments, the environmental control system and/or environmental control engine can capture room information (e.g., number and respective beds) from ADT and/or BMS systems automatically.

According to another embodiment, the environment control engine 104 can be configured to use state information (e.g., determined by the state component) to manage environment set points. Any changes in environment set points can be communicated to building management systems. In some embodiments, the environment set points can also be provided to environment control devices and/or environment management devices (e.g., air conditioning units, fans, heating units, lighting fixtures, etc.) either directly or through the BMS.

In one example, the environment devices can be configured to maintain a temperature for a room, based on a received temperature set point. In another example, the devices can control an airflow rate responsive to an environment set point. Other environment set points can also be provided to control other aspects of a room's environment. A lighting set point can be provided to alter behavior of lighting systems (e.g., turn off specific lights for an unoccupied state, motion detectors reconfigured to turn off lights based on reduced time periods of no motion, turn off lighting for a specific patient bed, etc).

In some embodiments, the environment set points can be entered on an environmental control system 100, using an administration component 110. Environment set points can be defined in groups for associated room occupancy states (e.g., occupied, unoccupied, one or more beds unoccupied, one or more beds occupied, etc). In some embodiments, the administration component 110 can be configured to display a user interface that displays room states and enables definition of environment set points for a respective state. For example, the interface can include a display for defining one or more parameters regarding specific room states. In one example, the various states can also be classified as major or minor depending on whether a patient is expected to return to a room. A major state change can include a patient discharge, transfer to another hospital, transfer to another ward, transfer to another room, among other options. State changes can be classified as major for occasions when a patient is not expected to return to a location. According to one embodiment, major state changes can be associated on the system with larger environment set point changes. The administration component 110 can be configured to allow submission of greater changes in environment control based on identification of a major state change.

In some embodiments, the administration component 110 can also be configured to enable definition of minor state changes associated with room occupancy when the patient is expected to return. For example, the environmental control engine 104 can capture schedule information from administration messages regarding a surgical procedure, a laboratory procedure, a therapy session, etc., which identify when a patient is expected to return. The administration component 110 can be configured to limit environment control parameters associated with a minor state change and/or the time period associated with the scheduled absence.

According to some embodiments, the environment control engine 104 can be configured to select environment set points communicated to a BMS based on determining if a state change is minor or major. In one example, the environment control engine 104 includes a control component 114 configured to select one or more environment set points (e.g., for temperature, airflow, humidity, lighting control, etc.) based on a type of state change and/or a scheduled period of absence.

According to some embodiments, the classification of major and minor state changes is not necessary to provide control over changes in environment set points. In some examples, the environment control system 100 and/or control engine 104 can be configured to project if and when a patient will return to an unoccupied room and select environment set points accordingly. In one implementation, environment set points can be defined based on a projected time of occupancy (e.g., determined by the state component 112). A control component 114 can be configured to select from the defined set points in response to the projected time of occupancy. Environment set points can be defined for any time period, including hours, minutes, etc. Where the state component 112 determines no occupancy is currently projected, the environment set points can be entered for that state as well.

In some implementations, the administration component 110 can display options for entering the environment set points by category (e.g., major or minor) and/or time period. In other embodiments, the user interface can display selections for major and minor states. As discussed, the major designation allows the system to define larger variations in the environment set points. For example, major designations are reserved for changes in occupancy that are likely to persist for some time. According to one embodiment, the environment control engine 104 can be configured to execute a major state change associated with the patient's room responsive to a patient discharge. The major state change can be communicated by the control component 114 to the BMS subsystem. The state change can be communicated with new set points to trigger the BMS to adjust environment settings.

In other embodiments, state changes can be associated with time periods, and a respective change in environment control can be tailored to the length of the associated time period. For example, an administration message can indicate that a patient is scheduled for a procedure (e.g., x-ray, MRI, CAT scan, etc.). The procedure can be associated with a schedule, a duration, etc. Based on an associated duration, the environment control engine 104 can be configured to select an environment set point (e.g., new temperature, or set point offset) by which to change an environmental control. In one example, the air temperature can be adjusted to minimize use of HVAC devices. In another example, the air exchange rate can be reduced. The air exchange rate refers to an airflow sufficient to move a volume of air equal to a space subject to the requirement from outside air to inside the subject space. Such an exchange can be very expensive in terms of the energy required to bring outside air in, and to provide the necessary heating, cooling, and/or humidity control to introduce it, for example, within a patient's room.

In some embodiments, the environment control engine 104 can be configured to calculate a maximum environment control change for the time period the room is expected to be unoccupied. Further, the control engine can determine an environmental change that allows for BMS subsystems to return the room under control back to a compliant state in time for the return of the patient.

In some implementations, environmental compliance is required, thus, the system can be configured to deliver environmental control message to return a room to compliance in advance of the patient's return. By resetting the environment control in advance of the patient's return, the system can insure compliance with any environmental requirements. In some embodiments, the environment control engine 104 can include a control component 114 configured to access a room's state, defined environment set points for the state, and generate environment control signals 106 to communicate, for example, to a building management system, and confirm compliance with environmental requirements. In one example, environmental compliance can be tracked by the building management system. The compliance information can be communicated to the environmental control system and tracked for analysis.

As discussed, an admission, discharge, and transfer system can be configured to communicate administration messages 102 to an environment control engine 104. In some embodiments, the environment control engine 104 can also receive administration messages from other systems. For example, a patient care facility can include a nurse call system and/or a bed management system. Known nurse call and bed management systems can be configured to manage patient assignments to rooms, provide for patients to undergo medical procedures, track patients between locations, and/or manage patient care. According to some embodiments, these systems can be configured to provide administration messages 102 to a control engine 104.

The environment control engine 104 can analyze the administration message to determine patient assignments to rooms, patient assignments to beds, scheduled procedures that take the patent out of a given among other options. The control engine can be configured to process patient location information and determine a state of a given room, and any number of beds within the given room. Responsive to the determine state, the environment control engine 104 can manage environment settings and output environment control signals 106, for example, to a BMS. According to one embodiment, integration with Nurse Call Systems and/or bed management system provides for addition control based on temporary absence from a room. In some embodiments, the additional systems provide information regarding patient's scheduled absence from a bed and/or room, enabling adjustments for any period where a room is not occupied. According to some embodiments, the environment control engine 104 is configured to provide for smaller adjustments relative to shorter periods of absence.

In some embodiments, the environment control engine 104 itself can be configured to perform the functions and operations discussed with respect to the various components rather than requiring any specific component. As discussed, the environment control engine 104 and any components can be implemented and the function discussed executed by an environmental control system (e.g., 100).

Shown in Fig. 2 is another embodiment of an environmental control system 200, which can be configured to execute an environment control engine (e.g., 104) or perform the operations discussed above with respect to the environmental control engine (e.g., 104) and any associated components. According to one embodiment, system 200 includes an environment control engine 202 configured to communicate with an administration system 201 and a building management system 206 over network 212. Network 212 can include a variety of network architectures, LAN, WAN, MAN, and can include, for example, connections to the Internet.

According to some embodiments, control engine 202 can be configured to receive administration messages from the administration system 201. The messages can be configured to provide information regarding patient location, patient room assignment, patient bed assignment, etc. The administration system 201 can include a number of subsystems. In one example, the administration system 201 can include an ADT subsystem 204 configured to manage admissions, transfers, and discharges of patients. The administration system 201 can also include a nurse call system 208 and/or a bed management system 210, which provide further information on patient location and/or room occupancy. For example, systems 208 and 210 can provide further detail on scheduled procedures (e.g., x-ray, physical therapy, examinations, etc.) establishing schedules for temporary absence from rooms, and can provide additional detail on occupancy for each bed in respective rooms.

According to one embodiment, environment control engine 202 can be configured to select offsets for environment control settings based on patient information communicated from the administration system 201. In one example, the control engine 202 can be configured to provide the greatest relative change in environmental control settings, in response to a persistent occupancy state change (e.g., patient discharge message) received from system 204. In another example, the control engine 202 can be configured to provide a smaller relative change in environmental control settings based on a temporary state changes (e.g., a patient who scheduled to return to an unoccupied room.

The environmental control engine 202 can be configured to provide control messages to a BMS system 206 to effect the new temperature settings and airflow rates, among other options. Control messages generated by the control engine 202 can also be configured to manage how the BMS system 206 controls lighting in a room, among other options. For example, lighting control messages can be tailored to specific areas of a room associated with one or more patient beds. Even though one bed within a room is occupied, control messages can limit lighting usage for lights associated with an unoccupied bed in the same room.

According to another embodiment, the control engine 202 can be configured to generate control messages for the BMS system 206 in a variety of formats. In one embodiment, the control engine 202 is configured to generate BACnet compliant messages and communicate the messages over network 212. Other embodiments can implement additional formats, for example, based on the BMS system 206 installed and/or the environment devices being managed.

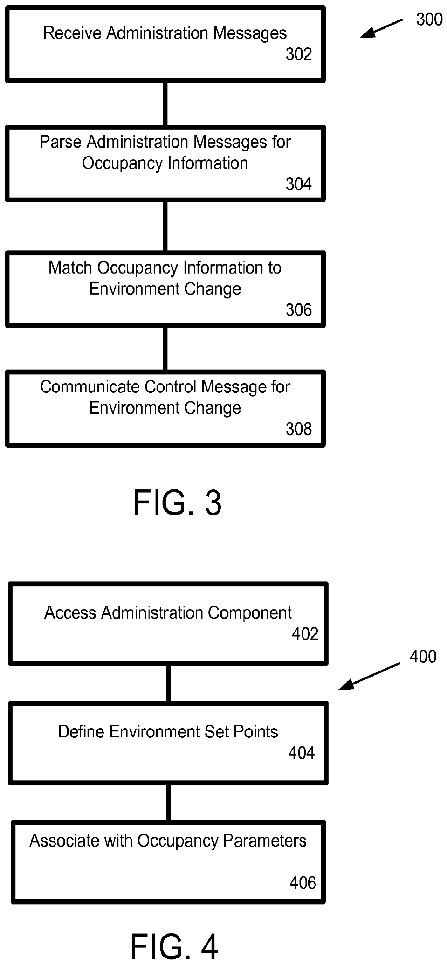

As discussed, a control engine and/or environmental control system can be configured to execute a variety of processes to implement patient occupancy based environmental settings. Fig. 3 illustrates an example process flow 300 for managing environment settings responsive to patient presence. The process 300 begins at 302 with receipt of administration messages. According to some embodiments, the administration messages are configured to include information regarding patient, patient locations, bed assignments, etc. The administration messages can include a variety of other information regarding a patient beyond location, room assignment, etc.

At 304, the administration messages can be analyzed to extract occupancy information. According to one example, the administration messages are analyzed to extract information regarding what patients are assigned to what rooms. In some further examples, the administration messages are analyzed to extract information regarding what patients are assigned to which beds within patient rooms.

The administration messages can be communicated from a variety of systems. For example, administration messages can be communicated by ADT systems, a nurse call system, and/or bed management systems. Each system can be configured to communicate administration messages in a respective format. Analysis of the messages at 304 can include any one or more of: identifying a messages format, identifying specific fields in a message, capturing patient identifiers, capturing location information associated with the patient identifier, capturing other locations associated with a patient identifier, and capturing any scheduling information associated with a patient identifier.

According to one embodiment, responsive to capturing information on patient location and/or room occupancy (e.g., at 304), the process 300 continues at 306 with matching occupancy information to an environment change. According to some embodiments, the occupancy information derived from administration messages (e.g., at 304) can indicate a permanent change in occupancy for one or more patients. For example, the patient can be discharged from a care facility, rendering the associated room unoccupied. Based on the change in occupancy determined, environment set points can be matched to the change in occupancy state. Multiple environment set points can be defined for a variety of changes in occupancy information. For example, a change in occupancy of one bed in a room having other occupants can be associated with changes in lighting settings, without any changes in temperature or airflow. Once remaining patients also leave the room, the information on occupancy can be matched to different environment set points. For example, a completely unoccupied room can be matched to environment set point changes that include reduced airflow rate, reduced temperature control, and changes in lighting settings.

The matching at 306 can also include identifying temporary occupancy changes. For example, the occupancy information determined at 304 can specify a time period after which a patient is expected to return the their respective room and/or bed. In some examples, the environment set points can be defined for specific time periods, and in other examples, can be defined based on whether the change in occupancy is temporary. In some embodiments, the matching at 306 can proceed with identifying a match based on a projected time period a room will be unoccupied. In one example, the changes in environment set points can be configured to allow a room to return to the same environment settings set during an occupied state by the expiration of the time period the room is schedule to be unoccupied.

Once new environment set points are identified, for example, by matching occupancy information to an environment change at 306, control messages can be generated and communicated to effect the change in environment control at 308. In one embodiment, control messages are generated, including any new environment set points or environment offsets, and are communicated to a BMS. The BMS can implement any changes in environment by controlling environment devices (e.g., heating units, air conditioning units, humidifiers, lighting controllers, etc.). In other embodiments, control messages can be generated and communicated directly to environment devices.

As discussed, matching of environment set points (e.g., 306) can include identifying temporary changes in occupancy and matching temporary changes to environment set points defined on temporary changes. Fig. 4 illustrates an example process 400 for defining changes in environment settings. The defined changes can be stored in a storage location (e.g., a database or other data repository) and accessed during execution of other processes (e.g., process 300 at 306) to manage environment settings. The process 400 begins at 402 with accessing an administration component.

According to one embodiment, the access to the administration component can include inputting a user name and password to determine authorization to access the administration component. In some implementations, the access to the administration component can be provided over a communication network (e.g., the internet). The communication network can be local to environment control systems, including any patient care location for which environmental control is desired. In other examples, the communication network can be connected to remote locations, and the access to the administration component can be provided through a web page or web portal.

At 404, environment set points are defined using the administration component. According to some embodiments, the administration component can display options for entering the environment set points by category (e.g., major or minor) and/or time period. For example, defining the environment set points at 404, can include specifying a change in temperature, airflow, humidity, which can also be associated with specification of occupancy parameters to match to the environment set points (e.g., at 406). In one instance, the occupancy parameters can be defined at 406 based on the occupancy state being temporary. For example, a patient may be associated with a scheduled absence and a scheduled return to their room. The period of time the room is scheduled to be unoccupied can be matched to environment set points and an associated occupancy parameters (e.g., unoccupied for at least 2 hours).

In another example, an occupancy parameter for a patient can be expected to persist. If a patient is discharged, typically there is no expectation that the patient will return, thus, the occupancy parameter can specify matching on a discharge order. The environment set points defined at 404 for a discharge parameter (e.g. set at 406) can be stored by a system and/or environment control engine executing process 400. According to some embodiments, the stored environment set points can be used by other processes executing on the system and/or engine to provide for environment control responsive to patient occupancy information.

### Example Computer System

As discussed above with regard to FIG. 1, various aspects and functions described herein may be implemented as specialized hardware or software components executing in one or more computer systems. There are many examples of computer systems that are currently in use. These examples include, among others, network appliances, personal computers, workstations, mainframes, networked clients, servers, media servers, application servers, database servers and web servers. Other examples of computer systems may include mobile computing devices, such as cellular phones and personal digital assistants, and network equipment, such as load balancers, routers and switches. Further, aspects may be located on a single computer system or may be distributed among a plurality of computer systems connected to one or more communications networks.

For example, various aspects and functions may be distributed among one or more computer systems configured to provide a service to one or more client computers, or to perform an overall task as part of a distributed system. Additionally, aspects may be performed on a client-server or multi-tier system that includes components distributed among one or more server systems that perform various functions. Consequently, examples are not limited to executing on any particular system or group of systems. Further, aspects and functions may be implemented in software, hardware or firmware, or any combination thereof. Thus, aspects and functions may be implemented within methods, acts, systems, system elements and components using a variety of hardware and software configurations, and examples are not limited to any particular distributed architecture, network, or communication protocol.

Referring to FIG. 5, there is illustrated a block diagram of a distributed computer system 500, in which various aspects and functions are practiced. As shown, the distributed computer system 500 includes one more computer systems that exchange information. More specifically, the distributed computer system 500 includes computer systems 502, 504 and 506. As shown, the computer systems 502, 504 and 506 are interconnected by, and may exchange data through, a communication network 508. For example, a environmental control system and/or environmental control engine can be implemented on 502, which can communicate with an ADT system implemented on 504, and a BMS system implemented on 506, which operate together to provide environmental control functions as discussed herein. In other embodiments, environmental control system and/or environmental control engine can include the ADT system, the BMS system, and the functions performed can be implement by 502 or distributed between 502-506.

In some embodiments, the network 508 may include any communication network through which computer systems may exchange data. To exchange data using the network 508, the computer systems 502, 504 and 506 and the network 508 may use various methods, protocols and standards, including, among others, Fibre Channel, Token Ring, Ethernet, Wireless Ethernet, Bluetooth, IP, IPV6, TCP/IP, UDP, DTN, HTTP, FTP, SNMP, SMS, MMS, SS7, JSON, SOAP, CORBA, REST and Web Services. To ensure data transfer is secure, the computer systems 502, 504 and 506 may transmit data via the network 508 using a variety of security measures including, for example, TLS, SSL or VPN. While the distributed computer system 500 illustrates three networked computer systems, the distributed computer system 500 is not so limited and may include any number of computer systems and computing devices, networked using any medium and communication protocol.

As illustrated in FIG. 5, the computer system 502 includes a processor 510, a memory 512, a bus 514, an interface 516 and data storage 518. To implement at least some of the aspects, functions and processes disclosed herein, the processor 510 performs a series of instructions that result in manipulated data. The processor 510 may be any type of processor, multiprocessor or controller. Some exemplary processors include commercially available processors such as an Intel Xeon, Itanium, Core, Celeron, or Pentium processor, an AMD Opteron processor, a Sun UltraSPARC or IBM Power5+ processor and an IBM mainframe chip. The processor 510 is connected to other system components, including one or more memory devices 512, by the bus 514.

The memory 512 stores programs and data during operation of the computer system 502. Thus, the memory 512 may be a relatively high performance, volatile, random access memory such as a dynamic random access memory (DRAM) or static memory (SRAM). However, the memory 512 may include any device for storing data, such as a disk drive or other non-volatile storage device. Various examples may organize the memory 512 into particularized and, in some cases, unique structures to perform the functions disclosed herein. These data structures may be sized and organized to store values for particular data and types of data.

Components of the computer system 502 are coupled by an interconnection element such as the bus 514. The bus 514 may include one or more physical busses, for example, busses between components that are integrated within the same machine, but may include any communication coupling between system elements including specialized or standard computing bus technologies such as IDE, SCSI, PCI and InfiniBand. The bus 514 enables communications, such as data and instructions, to be exchanged between system components of the computer system 502.

The computer system 502 also includes one or more interface devices 516 such as input devices, output devices and combination input/output devices. Interface devices may receive input or provide output. More particularly, output devices may render information for external presentation. Input devices may accept information from external sources. Examples of interface devices include keyboards, mouse devices, trackballs, microphones, touch screens, printing devices, display screens, speakers, network interface cards, etc. Interface devices allow the computer system 502 to exchange information and to communicate with external entities, such as users and other systems.

The data storage 518 includes a computer readable and writeable nonvolatile, or non-transitory, data storage medium in which instructions are stored that define a program or other object that is executed by the processor 510. The data storage 518 also may include information that is recorded, on or in, the medium, and that is processed by the processor 510 during execution of the program. More specifically, the information may be stored in one or more data structures specifically configured to conserve storage space or increase data exchange performance. The data storage can include specification of an occupancy state stored in association with a room, and/or bed with in the room, specification of environment set points associated with occupancy parameters. Further, the data storage can include time periods as an occupancy parameter, as well as a major or minor designation for any occupancy parameter.

The instructions stored in the date storage may be persistently stored as encoded signals, and the instructions may cause the processor 510 to perform any of the functions described herein. The medium may be, for example, optical disk, magnetic disk or flash memory, among other options. In operation, the processor 510 or some other controller causes data to be read from the nonvolatile recording medium into another memory, such as the memory 512, that allows for faster access to the information by the processor 510 than does the storage medium included in the data storage 518. The memory may be located in the data storage 518 or in the memory 512, however, the processor 510 manipulates the data within the memory, and then copies the data to the storage medium associated with the data storage 518 after processing is completed. A variety of components may manage data movement between the storage medium and other memory elements and examples are not limited to particular data management components. Further, examples are not limited to a particular memory system or data storage system.

Although the computer system 502 is shown by way of example as one type of computer system upon which various aspects and functions may be practiced, aspects and functions are not limited to being implemented on the computer system 502 as shown in FIG. 5. Various aspects and functions may be practiced on one or more computers having different architectures or components than that shown in FIG. 5. For instance, the computer system 502 may include specially programmed, special-purpose hardware, such as an application-specific integrated circuit (ASIC) tailored to perform a particular operation disclosed herein. While another example may perform the same function using a grid of several general-purpose computing devices running MAC OS System X with Motorola PowerPC processors and several specialized computing devices running proprietary hardware and operating systems.

The computer system 502 may be a computer system including an operating system that manages at least a portion of the hardware elements included in the computer system 502. In some examples, a processor or controller, such as the processor 510, executes an operating system. Examples of a particular operating system that may be executed include a Windows-based operating system, such as, Windows NT, Windows 2000 (Windows ME), Windows XP, Windows Vista, Windows 7 or 8 operating systems, available from the Microsoft Corporation, a MAC OS System X operating system available from Apple Computer, one of many Linux-based operating system distributions, for example, the Enterprise Linux operating system available from Red Hat Inc., a Solaris operating system available from Sun Microsystems, or a UNIX operating systems available from various sources. Many other operating systems may be used, and examples are not limited to any particular operating system.

The processor 510 and operating system together define a computer platform for which application programs in high-level programming languages are written. These component applications may be executable, intermediate, bytecode or interpreted code which communicates over a communication network, for example, the Internet, using a communication protocol, for example, TCP/IP. Similarly, aspects may be implemented using an object-oriented programming language, such as .Net, SmallTalk, Java, C++, Ada, C# (C-Sharp), Objective C, or Javascript. Other object-oriented programming languages may also be used. Alternatively, functional, scripting, or logical programming languages may be used.

Additionally, various aspects and functions may be implemented in a non-programmed environment, for example, documents created in HTML, XML or other format that, when viewed in a window of a browser program, can render aspects of a graphical-user interface or perform other functions. For example, an administration component can render an interface in a browser to enable definition of contamination risks.

Further, various examples may be implemented as programmed or non-programmed elements, or any combination thereof. For example, a web page may be implemented using HTML while a data object called from within the web page may be written in C++. Thus, the examples are not limited to a specific programming language and any suitable programming language could be used. Accordingly, the functional components disclosed herein may include a wide variety of elements, e.g., specialized hardware, executable code, data structures or data objects, that are configured to perform the functions described herein.

In some examples, the components disclosed herein may read parameters that affect the functions performed by the components. These parameters may be physically stored in any form of suitable memory including volatile memory (such as RAM) or nonvolatile memory (such as a magnetic hard drive). In addition, the parameters may be logically stored in a propriety data structure (such as a database or file defined by a user mode application) or in a commonly shared data structure (such as an application registry that is defined by an operating system). In addition, some examples provide for both system and user interfaces that allow external entities to modify the parameters and thereby configure the behavior of the components.

Having thus described several aspects of at least one example, it is to be appreciated that various alterations, modifications, and improvements will readily occur to those skilled in the art. For instance, examples disclosed herein may also be used in other contexts. Such alterations, modifications, and improvements are intended to be part of this disclosure, and are intended to be within the scope of the examples discussed herein. Accordingly, the foregoing description and drawings are by way of example only.

## Claims

1. A system for environmental control of patient care environments comprising:
a communication component configured to receive administration messages regarding patient location;
an interpretation component configured to capture information regarding patient location, patient room assignment, patient bed assignment, and/or any other information regarding scheduling of a patient's location associated with patient location from the administration messages;
a state component configured to determine a state for at least one room based at least in part on the information associated with patient location;
a generation component configured to generate an environment control message including at least one change to an environmental control parameter; and
at least one processor operatively connected to a memory, the at least one processor when executing is configured to:
determine information associated with patient location for a plurality of patient rooms;
establish at least one change to the environmental control parameter responsive to determining the information associated with patient location; and
communicate the environment control message to an environment management subsystem configured to implement at least one change to the environment control parameter, wherein the environment management subsystem is configured to manage environment devices configured to provide for at least one of heating, cooling, airflow, humidifying, and lighting,
**characterized in that** the state component is configured to determine the state according to a number of beds associated with the at least one room and **in that** the state component is further configured to determine occupancy of each bed within a room and enable environmental control based on occupancy status of each bed within a given room.

2. The system according to claim 1,
wherein the interpretation component is configured to capture the information associated with patient location from HL7 formatted messages.

3. The system according to claim 1, further comprising a control component configured to select the at least one change in the environmental control parameter based on the state for the at least one room.

4. The system according to claim 3, where the control component is configured to select the at least one change in the environmental control parameter based on a time period associated with the state.

5. The system according to claim 1, further comprising an administration subsystem configured to manage at least one of patient admission, discharge, and transfer.

6. A method for environmental control of patient care environments comprising:
receiving, by the computer system, administration messages regarding patient location ; and
capturing information regarding patient location, patient room assignment, patient bed assignment, and/or any other information regarding scheduling of a patient's location associated with patient location from the administration messages;
determining, by a computer system, information associated with patient location for a plurality of patient rooms;
determining a state for at least one room based at least in part on the information associated with patient location;
establishing, by the computer system, at least one change to an environmental control parameter responsive to determining the information associated with patient location;
generating, by the computer system, an environment control message including the at least one change to the environmental control parameter; and
communicating, by the computer system, the environment control message to a environment management subsystem configured to implement at least one change to the environment control parameter, wherein the environment management subsystem is configured to manage environment devices configured to provide for at least one of heating, cooling, airflow, humidifying, and lighting,
**characterized in that**
determining the state includes determining the state according to a number of beds associated with the at least one room and **in that** determining the state includes determining occupancy of each bed within a room and enable environmental control based on occupancy status of each bed within a given room.

7. The method according to claim 6, wherein capturing the information associated with patient location from the administration messages includes capturing the information associated with patient location from HL7 formatted messages.

8. The method according to claim 7, further comprising selecting the at least one change in the environmental control parameter based on the state for the at least one room.

## Patentansprüche

1. Ein System für die Umgebungssteuerung von Patientenversorgungsumgebungen, beinhaltend:
eine Kommunikationskomponente, die konfiguriert ist, um Administrationsnachrichten in Bezug auf den Aufenthaltsort eines Patienten zu empfangen;
eine Interpretationskomponente, die konfiguriert ist, um Informationen in Bezug auf den Aufenthaltsort eines Patienten, eine Patient-Zimmer-Zuordnung, eine Patient-Bett-Zuordnung und/oder beliebige andere Informationen in Bezug auf das Planen eines Aufenthaltsorts eines Patienten, die mit dem Aufenthaltsort eines Patienten assoziiert sind, aus den Administrationsnachrichten zu erfassen;
eine Statuskomponente, die konfiguriert ist, um einen Status für mindestens ein Zimmer zumindest teilweise auf der Basis der Informationen, die mit dem Aufenthaltsort eines Patienten assoziiert sind, zu bestimmen;
eine Generierungskomponente, die konfiguriert ist, um eine
Umgebungssteuerungsnachricht, die mindestens eine Änderung an einem Umgebungssteuerungsparameter umfasst, zu generieren; und
mindestens einen Prozessor, der betriebsfähig mit einem Speicher verbunden ist,
wobei der mindestens eine Prozessor beim Ausführen für Folgendes konfiguriert ist:
Bestimmen von Informationen, die mit dem Aufenthaltsort eines Patienten assoziiert sind, für eine Vielzahl von Patientenzimmern;
Festlegen mindestens einer Änderung an dem Umgebungssteuerungsparameter als Reaktion auf das Bestimmen der Informationen, die mit dem Aufenthaltsort eines Patienten assoziiert sind; und
Kommunizieren der Umgebungssteuerungsnachricht an ein Umgebungsverwaltungs-Untersystem, das konfiguriert ist, um mindestens eine Änderung an dem Umgebungssteuerungsparameter vorzunehmen, wobei das Umgebungsverwaltungs-Untersystem konfiguriert ist, um Umgebungsvorrichtungen zu verwalten, die konfiguriert sind, um mindestens eines von Heizen, Kühlen, Luftführung, Befeuchtung und Beleuchtung bereitzustellen,
**dadurch gekennzeichnet, dass** die Statuskomponente konfiguriert ist, um den Status gemäß einer Anzahl von Betten, die mit dem mindestens einen Zimmer assoziiert ist, zu bestimmen, und dass die Statuskomponente ferner konfiguriert ist, um die Belegung jedes Betts innerhalb eines Zimmers zu bestimmen und eine Umgebungssteuerung auf der Basis des Belegungsstatus jedes Betts innerhalb eines gegebenen Zimmers zu ermöglichen.

2. System gemäß Anspruch 1, wobei die Interpretationskomponente konfiguriert ist, um die Informationen, die mit dem Aufenthaltsort eines Patienten assoziiert sind, aus HL7-formatierten Nachrichten zu erfassen.

3. System gemäß Anspruch 1, ferner beinhaltend eine Steuerungskomponente, die konfiguriert ist, um die mindestens eine Änderung in dem Umgebungssteuerungsparameter auf der Basis des Status für das mindestens eine Zimmer auszuwählen.

4. System gemäß Anspruch 3, wobei die Steuerungskomponente konfiguriert ist, um die mindestens eine Änderung in dem Umgebungssteuerungsparameter auf der Basis einer Zeitspanne, die mit dem Status assoziiert ist, auszuwählen.

5. System gemäß Anspruch 1, ferner beinhaltend ein Administrations-Untersystem, das konfiguriert ist, um mindestens eines von einer Patientenaufnahme, einer Patientenentlassung und einem Patiententransfer zu verwalten.

6. Ein Verfahren für die Umgebungssteuerung von Patientenversorgungsumgebungen, beinhaltend:
Empfangen, durch das Computersystem, von Administrationsnachrichten in Bezug auf den Aufenthaltsort eines Patienten; und
Erfassen von Informationen in Bezug auf den Aufenthaltsort eines Patienten, eine Patient-Zimmer-Zuordnung, eine Patient-Bett-Zuordnung und/oder beliebigen anderen Informationen in Bezug auf das Planen eines Aufenthaltsorts eines Patienten, die mit dem Aufenthaltsort eines Patienten assoziiert sind, aus den Administrationsnachrichten; Bestimmen, durch ein Computersystem, von Informationen, die mit dem Aufenthaltsort eines Patienten assoziiert sind, für eine Vielzahl von Patientenzimmern;
Bestimmen eines Status für mindestens ein Zimmer zumindest teilweise auf der Basis der Informationen, die mit dem Aufenthaltsort eines Patienten assoziiert sind;
Festlegen, durch das Computersystem, mindestens einer Änderung an einem Umgebungssteuerungsparameter als Reaktion auf das Bestimmen der Informationen, die mit dem Aufenthaltsort eines Patienten assoziiert sind;
Generieren, durch das Computersystem, einer Umgebungssteuerungsnachricht, die die mindestens eine Änderung an dem Umgebungssteuerungsparameter umfasst; und
Kommunizieren, durch das Computersystem, der Umgebungssteuerungsnachricht an ein Umgebungsverwaltungs-Untersystem, das konfiguriert ist, um mindestens eine Änderung an dem Umgebungssteuerungsparameter vorzunehmen, wobei das Umgebungsverwaltungs-Untersystem konfiguriert ist, um Umgebungsvorrichtungen zu verwalten, die konfiguriert sind, um mindestens eines von Heizen, Kühlen, Luftführung, Befeuchtung und Beleuchtung bereitzustellen,
**dadurch gekennzeichnet, dass**
das Bestimmen des Status das Bestimmen des Status gemäß einer Anzahl von Betten, die mit dem mindestens einen Zimmer assoziiert ist, umfasst und dass das Bestimmen des Status das Bestimmen der Belegung jedes Betts innerhalb eines Zimmers und das Ermöglichen einer Umgebungssteuerung auf der Basis des Belegungsstatus jedes Betts innerhalb eines gegebenen Zimmers umfasst.

7. Verfahren gemäß Anspruch 6, wobei das Erfassen der Informationen, die mit dem Aufenthaltsort eines Patienten assoziiert sind, aus den Administrationsnachrichten das Erfassen der Informationen, die mit dem Aufenthaltsort eines Patienten assoziiert sind, aus HL7-formatierten Nachrichten umfasst.

8. Verfahren gemäß Anspruch 7, ferner beinhaltend das Auswählen der mindestens einen Änderung in dem Umgebungssteuerungsparameter auf der Basis des Status für das mindestens eine Zimmer.

## Revendications

1. Un système de contrôle environnemental d'environnements de soins aux patients comprenant:
un composant de communication configuré pour recevoir des messages d'administration concernant un emplacement de patient ;
un composant d'interprétation configuré pour capturer des informations concernant un emplacement de patient, l'attribution d'une chambre à un patient, l'attribution d'un lit à un patient, et/ou toutes autres informations concernant la planification de l'emplacement d'un patient associées à un emplacement de patient à partir des messages d'administration ;
un composant d'état configuré pour déterminer un état pour au moins une chambre sur la base, au moins en partie, des informations associées à un emplacement de patient ;
un composant de génération configuré pour générer un message de contrôle d'environnement incluant au moins un changement à un paramètre de contrôle environnemental ; et
au moins un processeur connecté de manière fonctionnelle à une mémoire, l'au moins un processeur étant configuré pour, en exécution :
déterminer des informations associées à un emplacement de patient pour une pluralité de chambres de patient ;
établir au moins un changement au paramètre de contrôle environnemental en réponse à la détermination des informations associées à un emplacement de patient ; et
communiquer le message de contrôle d'environnement à un sous-système de gestion d'environnement configuré pour mettre en oeuvre au moins un changement au paramètre de contrôle d'environnement, le sous-système de gestion d'environnement étant configuré pour gérer des dispositifs d'environnement configurés afin de pourvoir à au moins un élément parmi : chauffage, refroidissement, écoulement d'air, humidification, et éclairage,
**caractérisé en ce que** le composant d'état est configuré pour déterminer l'état en fonction d'un nombre de lits associés à l'au moins une chambre et **en ce que** le composant d'état est configuré en outre pour déterminer l'occupation de chaque lit à l'intérieur d'une chambre et mettre en route un contrôle environnemental sur la base du statut d'occupation de chaque lit à l'intérieur d'une chambre donnée.

2. Le système selon la revendication 1, dans lequel le composant d'interprétation est configuré pour capturer les informations associées à un emplacement de patient à partir de messages formatés HL7.

3. Le système selon la revendication 1, comprenant en outre un composant de contrôle configuré pour sélectionner l'au moins un changement dans le paramètre de contrôle environnemental sur la base de l'état pour l'au moins une chambre.

4. Le système selon la revendication 3, où le composant de contrôle est configuré pour sélectionner l'au moins un changement dans le paramètre de contrôle environnemental sur la base d'un laps de temps associé à l'état.

5. Le système selon la revendication 1, comprenant en outre un sous-système d'administration configuré pour gérer au moins un élément parmi l'admission, la sortie, et le transfert d'un patient.

6. Un procédé de contrôle environnemental d'environnements de soins aux patients comprenant :
la réception, par le système informatique, de messages d'administration concernant un emplacement de patient ; et
la capture d'informations concernant un emplacement de patient, l'attribution d'une chambre à un patient, l'attribution d'un lit à un patient, et/ou toutes autres informations concernant la planification de l'emplacement d'un patient associées à un emplacement de patient à partir des messages d'administration ;
la détermination, par un système informatique, d'informations associées à un emplacement de patient pour une pluralité de chambres de patient ;
la détermination d'un état pour au moins une chambre sur la base, au moins en partie, des informations associées à un emplacement de patient ;
l'établissement, par le système informatique, d'au moins un changement à un paramètre de contrôle environnemental en réponse à la détermination des informations associées à un emplacement de patient ;
la génération, par le système informatique, d'un message de contrôle d'environnement incluant l'au moins un changement au paramètre de contrôle environnemental ; et
la communication, par le système informatique, du message de contrôle d'environnement à un sous-système de gestion d'environnement configuré pour mettre en oeuvre au moins un changement au paramètre de contrôle d'environnement, le sous-système de gestion d'environnement étant configuré pour gérer des dispositifs d'environnement configurés afin de pourvoir à au moins un élément parmi : chauffage, refroidissement, écoulement d'air, humidification, et éclairage,
**caractérisé en ce que**
la détermination de l'état inclut la détermination de l'état en fonction d'un nombre de lits associés à l'au moins une chambre et **en ce que** la détermination de l'état inclut la détermination de l'occupation de chaque lit à l'intérieur d'une chambre et la mise en route d'un contrôle environnemental sur la base du statut d'occupation de chaque lit à l'intérieur d'une chambre donnée.

7. Le procédé selon la revendication 6, dans lequel la capture des informations associées à un emplacement de patient à partir des messages d'administration inclut la capture des informations associées à un emplacement de patient à partir de messages formatés HL7.

8. Le procédé selon la revendication 7, comprenant en outre la sélection de l'au moins un changement dans le paramètre de contrôle environnemental sur la base de l'état pour l'au moins une chambre.
